# EUROPÄISCHE PATENTSCHRIFT

(11) **EP 1 264 606 B1**
(45) Veröffentlichungstag und Bekanntmachung des Hinweises auf die Patenterteilung: **02.07.2008**
(21) Anmeldenummer: 02009218.5
(22) Anmeldetag: 25.04.2002
(51) Int. Cl.: A61L 27/32

(54) **Apatitbeschichteter metallischer Werkstoff, Verfahren zu dessen Herstellung sowie Verwendung**
Apatite coating of metallic materials, process and use thereof
Revêtements d'apatite pour substrats métalliques, procédés de fabrication, et utilisation

(30) Priorität: 06.06.2001 DE 10128259
(43) Veröffentlichungstag der Anmeldung: 11.12.2002
(73) Patentinhaber: Biomet Merck GmbH, 3216 Ried b. Kerzers (CH)
(72) Erfinder: Kotte, Bernd, 01279 Dresden (DE); Hofinger, Jürgen, Dr., 01187 Dresden (DE); Hebold, Tanja, 01169 Dresden (DE)
(74) Vertreter: Gross, Felix

(56) Entgegenhaltungen:
- US-A- 5 205 921
- US-A- 5 759 376

## Beschreibung

Die Erfindung betrifft einen neuen apatitbeschichteten metallischen Werkstoff mit verbesserter Oberflächenqualität und Biokompatibilität, ein Verfahren zu dessen Herstellung sowie die Verwendung des Werkstoffes für Knochenimplantate, insbesondere Zahnimplantate, künstliche Gelenke und Befestigungsmaterial für die Unfallchirurgie (Osteosynthesematerial).

Es ist bekannt, dass mit Calciumphosphat, insbesondere mit dem Knochenmineral Hydroxylapatit (HAP (Ca₁₀(PO₄)6OH)), beschichtete Implantate besser einwachsen. Für die Beschichtung von Implantaten mit Calciumphosphat kommen unterschiedliche Verfahren zum Einsatz, wie z. B. Plasmaspritzen, SolGel-Verfahren, Elektrophorese, elektrochemisch gestützte Abscheidung.

Die elektrochemisch gestützte Abscheidung hat Vorteile gegenüber anderen Verfahren aufgrund der Möglichkeit der Erzeugung von gleichmäßigen (auch bei sehr rauhen Oberflächen) sowie dünnen Schichten, der Möglichkeit zur gezielten Steuerung der abzuscheidenden Phasen über elektrische Parameter und ist kostengünstiger in der Herstellung.

Bei der elektrochemisch gestützten Abscheidung fällt Calciumphosphat an der Kathode, die das Implantat bildet, aus. Die entstehende Schicht ist sehr porös und lässt sich daher leicht von der Oberfläche entfernen. Es gibt verschiedene Möglichkeiten, die Porosität zu verringern.

Nach US 3892648 wird eine Emulation aus Knochenpuder und Kollagen elektrochemisch auf das metallische Implantat aufgetragen und damit durch das Kollagen eine stärkere Haftfestigkeit erreicht.

In DE 19504386 A1 wird die abgeschiedene Calciumphosphat-Schicht gradiert mit der Metalloberfläche verbunden. Calciumphosphat-Kristalle werden von einer wachsenden Oxidschicht umschlossen. Nachteil dieses Verfahrens ist: Es funktioniert nur mit Implantaten aus Titan bzw. Titanlegierungen. Die Porosität der Beschichtung wird nicht verringert und die mechanischen Eigenschaften damit nicht verbessert.

Bei Patent US 5458863 wird mit einer elektrochemisch gestützten Abscheidung zunächst eine Brushit-Schicht erzeugt, die anschließend bei Temperaturen zwischen 20 und 100 °C zu Hydroxylapatit umgewandelt wird. Die Haftfestigkeit zwischen Schicht und Substrat wird durch regelmäßiges Entfernen von Gasblasen an der Substratoberfläche während der Beschichtung verbessert. Nachteilig ist bei diesem Verfahren, dass der Umwandlungsprozess ca. 36 Stunden dauert. Bei Temperaturen von 750 ° C bilden sich nach WO 9813539 aus der elektrochemisch gestützten Abscheidung einer Calciumphosphat-Phase Hydroxylapatit-Kristalle und die Haftfestigkeit wird verbessert. Hier sind vor allem die hohen Temperaturen als besonders nachteilig zu erwähnen. Im Patent US 5205921 wird nach dem elektrolytischen Abscheiden die Haftfestigkeit der erzeugten Schicht mittels Ultraschallverfahren im Methanolbad verbessert. Die Methode basiert darauf, dass Kristallite mit geringer Haftfestigkeit zum Substrat durch den Einfluss von Ultraschall wieder abgelöst werden.

Nach wie vor besteht ein hoher Bedarf an Implantatwerkstoffen mit verbesserter Oberfläche und Kompatibilität zum biologischen System.

Aufgabe der Erfindung ist deshalb ein apatitbeschichteter metallischer Werkstoff mit verringerter Porosität und verbesserter Haftfestigkeit.

Erfindungsgemäß wird die Aufgabe durch einen apatitbeschichteten metallischen Werkstoff gelöst, bei dem die Beschichtung aus einer dichten Bedeckung von Hydroxylapatit-Kristallen mit einer Nadellänge im Bereich von 200 bis 300 nm oder amorphen Calciumphosphatkugeln mit einem Durchmesser im Bereich von 35 bis 200 nm mit einer Schichtdicke > 1 um besteht und die Beschichtung eine spezifische Oberfläche kleiner 15 qm/g aufweist.

Der metallische Werkstoff besteht aus Titan oder Titanlegierungen, CoCrMo-Legierungen oder rostfreien Stählen.

Erfindungsgemäß wird der neue apatitbeschichtete Werkstoff durch ein elektrochemisch gestütztes Verfahren mit einer vom Metallwerkstoff gebildeten Substratelektrode und einer Gegenelektrode gelöst, bei dem als Elektrolyt eine wässrige Lösung mit Calciumund Phosphationen verwendet wird.

Erfindungsgemäß erfolgt die Beschichtung durch kathodische Polarisierung in mehreren aufeinander folgenden Prozesszyklen. Ein Prozesszyklus besteht aus kathodischer Polarisierung in ein oder mehreren aufeinanderfolgenden Stufen mit gleich oder verschieden hohen konstanten Stromdichten, und sich daran anschließender Spülungsund/oder Trocknungsphase.

Das Konzentrationsverhältnis von Calcium- und Phosphationen im Elektrolyten entspricht dem von Hydroxylapatit.

Durch das erfindungsgemäße Verfahren erfolgt eine Verringerung der Porosität dadurch, dass der Prozess in mehreren Zyklen mit elektrochemischer Calciumphosphatabscheidung und anschließender Spülung und/oder Trocknung mehrmals wiederholt wird.

Elektrochemisch werden auf dem metallischen Werkstoff Hydroxylapatit oder deren Vorstufen (Amorphes Calciumphosphat (ACP) / Mischzustände ACP-HAP) abgeschieden. Die Größe der Hydroxylapatit-Kristalle liegt zwischen 200 bis 300 nm Die amorphen Kugeln können in ihrem Durchmesser im Bereich von 35 bis 200 nm variiert werden. Die verdichteten Schichten werden durch Wechsel zwischen kurzen Beschichtungsphasen und sich daran anschließenden Spülungs- und/oder Trocknungsphasen erreicht. Die Trocknung erfolgt bei Raumtemperatur. Bei der Trocknung wird den porösen Schichten die verbrauchte Elektrolytflüssigkeit entzogen. Beim nächsten Eintauchen füllen sich die Hohlräume mit frischer Elektrolytflüssigkeit. Damit erfolgt auch in den Hohlräumen wieder eine elektrochemisch gestützte Abscheidung von Calciumphosphat-Phasen. Außerdem wird der Werkstoffkörper während der Beschichtungs- und Trocknungsphasen laufend bewegt, um eine gleichmäßige Beschichtung, auch bei speziell gestalteten Werkstoffkörpern und sehr rauhen/porösen Oberflächen zu bekommen.

In einer Ausgestaltung des erfindungsgemäßen Verfahrens erfolgt die kathodische Polarisation bei einer Konstantstromdichte von 0,5 mA/cm² bis 20 mA/cm² oder in den einzelnen Prozesszyklen bei unterschiedlichen Stromdichten, wobei die Stromdichte in den Folgezyklen verringert wird.

Die Erfindung beinhaltet auch die Verwendung der neuen apatitbeschichteten metallischen Werkstoffe für die Herstellung von Implantaten, insbesondere Zahn- und Gelenkimplantaten sowie Material zur Stabilisierung des Knochens bei Frakturen (Osteosynthesematerial).

Anhand nachfolgender Ausführungsbeispiele wird die Erfindung näher erläutert:

### Ausführungsbeispiel 1

Beschichtungsprobe: Zylinder aus Titanlegierung (TiA16V4), 10 mm Durchmesser, 46 mm lang, mit SIC-Papier, grit 1200, geschliffen.

Diese Probe wurde vor der Beschichtung mit Ultraschall in Ethanol gereinigt, mit deoinisiertem Wasser abgespült, über Luftstrom getrocknet, anschließend an beiden Enden mit einer Halterung incl. Kontakteinrichtung aus Deguform Siliconmasse abgedeckt. Die zu beschichtende Fläche betrug 6,28 cm².

Die Elektrolytflüssigkeit wurde hergestellt aus 5 Liter deionisiertem Wasser mit eingeführtem 2,455 g CaCl₂*2H₂O und 1,15 g NH₄H₂PO₄, was einem Ca/P Verhältnis von 1,67 entspricht. Die über Thermostat geregelte Temperatur der Elektrolytflüssigkeit betrug 37 °C. Der pH-Wert wurde mit einer NH₄OH-Lösung auf 6,45 eingestellt.

Die Probe ist als Kathode gepolt; als Anode wurden Platinnetzelektroden verwendet. Die Beschichtung erfolgte in 10 Zyklen. Ein Zyklus umfasste:

Fünfminütige kathodische Polarisation mit einem Konstantstrom von 63 mA, anschließend 1 Minute Spülen in deionisiertem Wasser und danach 5 Minuten Trocknen mit Lüfter.

Ergebnisse:

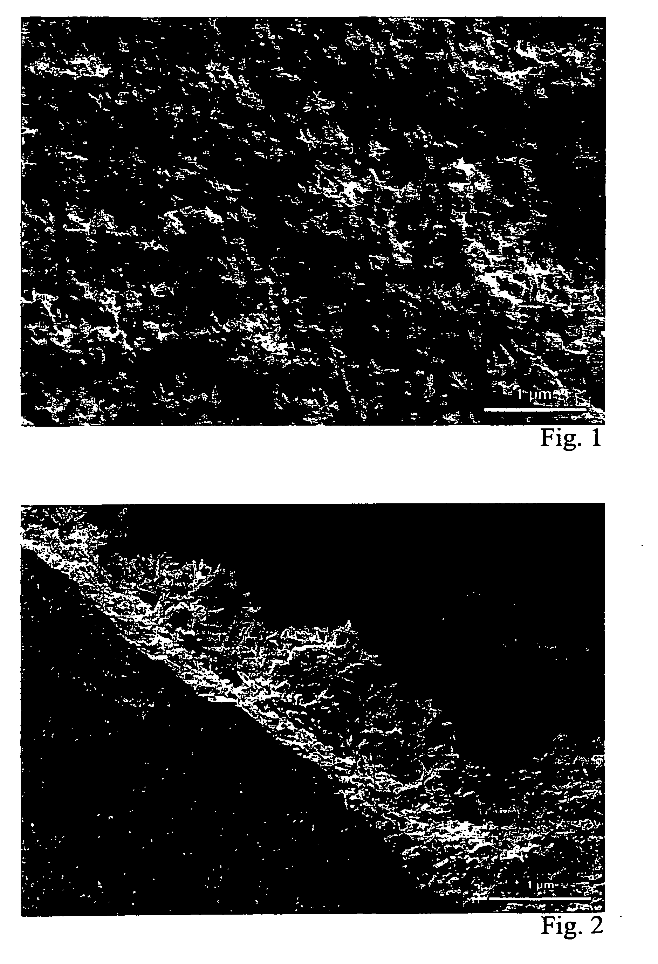

**Fig.1** zeigt eine REM-Aufnahme der nach Ausführungsbeispiel 1 erhaltenen Apatitbeschichtung auf TiA16V4. Makroskopisch erscheint die Schicht gleichmäßig weiß und haftet gut. Die Untersuchung am REM zeigt eine geschlossene Schicht mit apatitförmigen Nadeln von ca. 200 - 300 nm Länge. Die Energiedispersive Röntgenanalyse ergibt ein Ca/P Verhältnis von 1,67, das entspricht Hydroxylapatit. Die BET-Analyse nach DIN 66 131 ergibt eine spezifische Oberfläche von 9,25 Quadratmeter pro Gramm. Im Vergleich dazu wird bei einer nicht verdichteten Abscheidung eine spezifische Oberfläche von ca. 60 Quadratmeter/Gramm erreicht. Durch Einritzen und Schräganalyse am REM wurde die Schichtdicke mit ca. 1,8 um vermessen.

Eine IR-Spektroskopische Analyse (FTIR) bestätigt, dass es sich bei der Beschichtung um Hydroxylapatit handelt. Die IR-Spektren von Hydroxylapatitpulvern der Firma Merck und der beschichteten Probe haben gleiche Absorptionsbanden.

**Fig. 2** zeigt eine REM-Aufnahme der nach Ausführungsbeispiel 1 erhaltenen Apatitbeschichtung auf TiA16V4, die mechanisch von der Substratoberfläche abgelöst wurde. Auf der Beschichtungsunterseite (substratseitig) ist ein Bereich hoher Dichte zu erkennen, der in Richtung Schichtoberfläche abnimmt.

### Ausführungsbeispiel 2

Gleiche Probengröße, Beschichtung und Ergebnisse wie bei Ausführungsbeispiel 1. Aber das Probenmaterial ist die Legierung CoCr28Mo.

### Ausführungsbeispiel 3

Gleiche Probengröße, Beschichtung wie bei Ausführungsbeispiel 1. Aber innerhalb der Prozesszyklen wurde die Polarisation in zwei Stufen mit folgenden Stromdichten durchgeführt:

1 Min. mit 75 mA, 4 Minuten mit 50 mA.

Ergebnis: Nadellänge ca. 200 - 300 nm, noch dichtere Packung.

### Ausführungsbeispiel 4

Gleiche Probengröße, Beschichtung wie bei Ausführungsbeispiel 1. Aber innerhalb der 5 Minuten kathodischer Polarisation wurde im Zyklus 1, 3 und 8 der Strom wie folgt geändert: 1 Min. mit 63 mA, 4 Min mit 5,6 mA. Bei den Zyklen 2, 4, 5, 6, 7, 9, 10 betrug der Strom 5,6 mA.

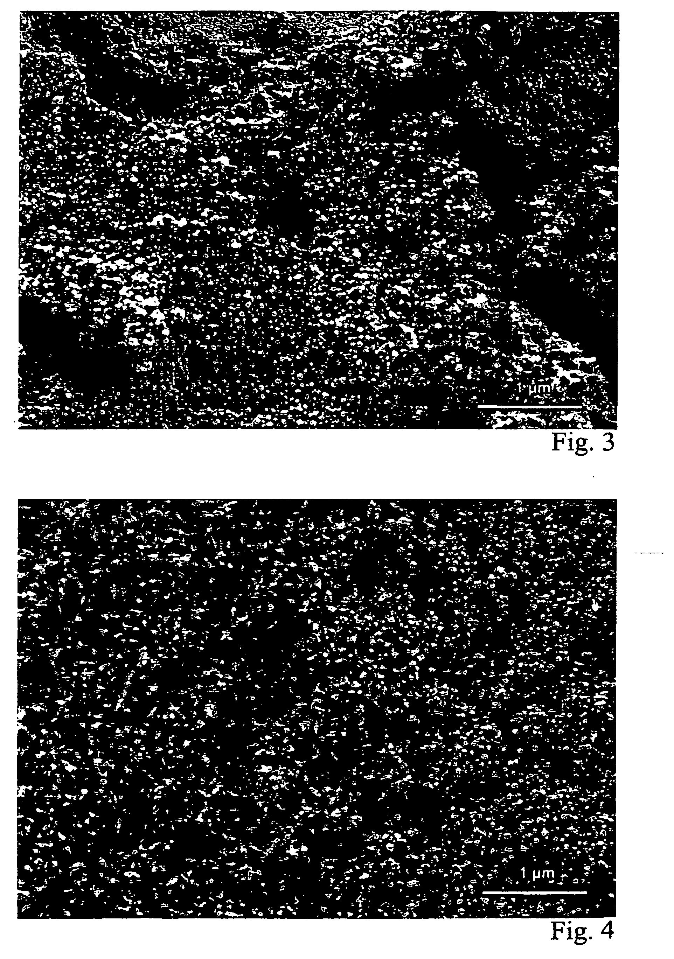

**Fig. 3** zeigt eine REM-Aufnahme der nach Ausführungsbeispiel 3 erhaltenen Beschichtung. Makroskopisch erscheint die Schicht gleichmäßig weiß und haftet gut. Die Untersuchung am REM zeigt eine geschlossene Schicht mit Calciumphosphatkugeln von ca. 50 bis 150 nm Durchmesser. Durch Einritzen und Schräganalyse am REM wurde die Schichtdicke mit ca. 1,8 µm vermessen.

### Ausführungsbeispiel 5

Gleiche Probengröße, Beschichtung wie bei Ausführungsbeispiel 1, aber in 25 Zyklen. Ein Zyklus umfasst: 1 Min. kathodische Polarisation mit 63 mA, 1 Min. mit 50 mA.

Ergebnis: Mischzustand (ACP-Kugeln und HAP-Nadeln). Siehe Fig. 4

**Fig. 4** zeigt eine REM-Aufnahme der nach Ausführungsbeispiel 5 erhaltenen Beschichtung. Makroskopisch erscheint die Schicht gleichmäßig weiß und haftet gut. Die Untersuchung am REM zeigt eine geschlossene Schicht mit Calciumphosphatkugeln und Hydroxylapatitnadeln.

## Patentansprüche

1. Apatitbeschichteter metallischer Werkstoff,
**dadurch gekennzeichnet,**
**dass** die Beschichtung aus einer dichten Bedeckung von Hydroxylapatitkristallen mit einer Nadellänge von 200 bis 300 nm oder amorphen Calciumphosphatkugeln mit einem Durchmesser von 35 bis 200 nm besteht und die Beschichtung eine spezifische Oberfläche kleiner 15 m²/g aufweist.

2. Werkstoff nach Anspruch 1, **dadurch gekennzeichnet, dass** der metallische Werkstoff aus Titan oder Titanlegierungen, CoCrMo-Legierungen oder rostfreien Stählen besteht.

3. Verfahren zur Herstellung eines apatitbeschichteten metallischen Werkstoffes durch ein elektrochemisch gestütztes Verfahren mit einer vom Metallwerkstoff gebildeten Substratelektrode und einer Gegenelektrode, bei dem als Elektrolyt eine wässrige Lösung mit Calcium- und Phosphationen verwendet wird, **dadurch gekennzeichnet, dass** eine kathodische Polarisierung in mehreren aufeinander folgenden Prozesszyklen erfolgt, wobei eine Prozesszyklus aus kathodischer Polarisierung in ein oder mehreren aufeinanderfolgenden Stufen mit gleich oder verschieden hohen konstanten Stromdichten und sich daran anschließender Spülungs- und/oder Trocknungsphase besteht.

4. Verfahren nach Anspruch 3, **dadurch gekennzeichnet, dass** das Konzentrationsverhältnis von Calcium- und Phosphationen im Elektrolyten dem von Hydroxylapatit entspricht.

5. Verfahren nach Anspruch 3 oder 4, **dadurch gekennzeichnet, dass** der Werkstoff während der Prozesszyklen laufend gedreht wird.

6. Verfahren nach einem der Ansprüche 3 bis 5, **dadurch gekennzeichnet, dass** die kathodische Polarisation bei einer Konstantstromdichte von 0,5 mA/cm² bis 20 mA/cm² oder in den einzelnen Prozesszyklen bei unterschiedlichen Stromdichten erfolgt, wobei die Stromdichte in den Folgezyklen verringert wird.

7. Verwendung eines Werkstoffes nach Anspruch 1 oder 2 zur Herstellung von Knochenimplantaten, Zahnimplantaten, künstlichen Gelenken oder Befestigungsmaterial für die Unfallchirurgie (Osteosynthesematerial).

8. Verwendung eines nach einem der Ansprüche 3 bis 6 beschichteten Werkstoffes zur Herstellung von Knochenimplantaten, Zahnimplantaten, künstliche Gelenken oder Befestigungsmaterial fiir die Unfallchirurgie (Osteosynthesematerial).

## Claims

1. Apatite-coated metallic material, **characterized in that** the coating consists of a dense covering of hydroxyapatite crystals having a needle length of from 200 to 300 nm or amorphous calcium phosphate spheres having a diameter of from 35 to 200 nm and the coating has a specific surface area of less than 15 m²/g.

2. Material according to Claim 1, **characterized in that** the metallic material consists of titanium or titanium alloys, CoCrMo alloys or stainless steels.

3. Process for the preparation of an apatite-coated metallic material by an electrochemically assisted process using a substrate electrode formed by the metallic material and a counterelectrode in which, as electrolyte, an aqueous solution containing calcium and phosphate ions is used, **characterized in that** a cathodic polarization takes place in a number of successive process cycles, a process cycle consisting of cathodic polarization in one or more successive stages having identical or different high constant current densities and a rinsing and/or drying phase following thereon.

4. Process according to Claim 3, **characterized in that** the concentration ratio of calcium and phosphate ions in the electrolyte corresponds to that of hydroxyapatite.

5. Process according to Claim 3 or 4, **characterized in that** the material is constantly turned during the process cycles.

6. Process according to one of Claims 3 to 5, **characterized in that** the cathodic polarization is carried out at a constant current density of 0.5 mA/cm² to 20 mA/cm² or takes place in the individual process cycles at different current densities, the current density being decreased in the subsequent cycles.

7. Use of a material according to Claim 1 or 2 for the preparation of bone implants, dental implants, artificial joints or fixative material for accident surgery (osteosynthesis material).

8. Use of a material coated according to one of Claims 3 to 6 for the preparation of bone implants, dental implants, artificial joints or fixative material for accident surgery (osteosynthesis material).

## Revendications

1. Matériau métallique revêtu d'apatite, **caractérisé en ce que** le revêtement est constitué par un recouvrement dense de cristaux d'hydroxyapatite avec une longueur d'aiguilles de 200 à 300 nm ou de billes de phosphate de calcium amorphes avec un diamètre de 35 à 200 nm et le revêtement présente une surface spécifique inférieure à 15 m²/g.

2. Matériau selon la revendication 1, **caractérisé en ce que** le matériau métallique est constitué par du titane ou des alliages de titane, des alliages de CoCrMo ou des aciers inoxydables.

3. Procédé pour la préparation d'un matériau métallique revêtu d'apatite par un procédé soutenu par voie électrochimique avec une électrode de substrat formée par le matériau métallique et une contre-électrode, dans lequel on utilise, comme électrolyte, une solution aqueuse avec des ions de calcium et de phosphate, **caractérisé en ce qu'**il se produit une polarisation cathodique dans plusieurs cycles de processus consécutifs, un cycle de processus étant constitué par une polarisation cathodique dans une ou plusieurs étapes consécutives, avec des densités de courant constantes élevées identiques ou différentes et des phases de rinçage et/ou de séchage consécutives.

4. Procédé selon la revendication 3, **caractérisé en ce que** le rapport des concentrations en ions de calcium et de phosphate dans l'électrolyte correspond à celui de l'hydroxyapatite.

5. Procédé selon la revendication 3 ou 4, **caractérisé en ce que** le matériau est tourné en continu pendant les cycles de processus.

6. Procédé selon l'une quelconque des revendications 3 à 5, **caractérisé en ce que** la polarisation cathodique est réalisée à une densité de courant constante de 0,5 mA/cm² à 20 mA/cm² ou, dans les différents cycles de processus, à des densités de courant différentes, la densité de courant étant diminuée dans les cycles successifs.

7. Utilisation d'un matériau selon la revendication 1 ou 2 pour la réalisation d'implants osseux, d'implants dentaires, d'articulations artificielles ou de matériau de fixation pour la traumatologie (matériau d'ostéosynthèse).

8. Utilisation d'un matériau revêtu selon l'une quelconque des revendications 3 à 6 pour la réalisation d'implants osseux, d'implants dentaires, d'articulations artificielles ou de matériau de fixation pour la traumatologie (matériau d'ostéosynthèse).
